# FASCICULE DE BREVET EUROPEEN

(11) **EP 4 589 289 B1**
(45) Date de publication et mention de la délivrance du brevet: **09.09.2026**
(21) Numéro de dépôt: 25152371.8
(22) Date de dépôt: 16.01.2025
(51) Int. Cl.: G01N 27/404, G01N 27/413, G01N 27/416, G01N 33/00

(54) **PROCÉDÉ DE PRÉPARATION D'UN CAPTEUR DE DIOXYDE DE CARBONE, LEDIT CAPTEUR ET SES UTILISATIONS**
VERFAHREN ZUR HERSTELLUNG EINES KOHLENDIOXIDSENSORS, KOHLENDIOXIDSENSOR UND VERWENDUNGEN DAVON
METHOD FOR PREPARING A CARBON DIOXIDE SENSOR, SAID SENSOR AND USES THEREOF

(30) Priorité: 17.01.2024 FR 2400465
(43) Date de publication de la demande: 23.07.2025
(73) Titulaire: COMMISSARIAT À L'ÉNERGIE ATOMIQUE ET AUX ÉNERGIES ALTERNATIVES, 75015 Paris (FR)
(72) Inventeur: CHATARD, Charles, 38054 GRENOBLE Cedex 09 (FR)
(74) Mandataire: Santarelli

(56) Documents cités:
- JP-A- 2001 208 722
- US-A1- 2008 268 421
- KOJIMA S ET AL: "Microanalysis system for pO2, pCO2, and pH constructed with stacked modules", IEEE SENSORS JOURNAL, IEEE, USA, vol. 5, no. 5, 1 October 2005 (2005-10-01), pages 1120 - 1126, XP011138641, ISSN: 1530-437X, DOI: 10.1109/JSEN.2005.853596

## Description

### DOMAINE TECHNIQUE

La présente invention appartient au domaine des capteurs électrochimiques utiles pour la détermination de paramètres chimiques, notamment au domaine des capteurs de dioxyde de carbone dissous ou volatil.

La présente invention propose un procédé permettant de préparer un capteur de dioxyde de carbone et notamment un capteur de dioxyde de carbone miniaturisé.

La présente invention concerne également un tel capteur de dioxyde de carbone et ses différentes utilisations.

### ÉTAT DE LA TECHNIQUE ANTÉRIEURE

La détection et/ou le suivi du dioxyde de carbone est primordiale pour de nombreuses applications telles que, par exemple, la surveillance des conditions environnementales, l'évaluation des niveaux de pollution de l'air, la compréhension de processus physiologiques ou encore la régulation de procédés de production biopharmaceutique, ...). De nombreuses approches technologiques ont été développées pour détecter et mesurer le dioxyde de carbone, chacune ayant ses propres avantages et limites.

Parmi les méthodes conventionnelles pour déterminer la concentration de dioxyde de carbone, figurent la spectrométrie infrarouge et les capteurs électrochimiques basés sur des électrolytes liquides ou solides. La détection infrarouge est une technique largement utilisée qui repose sur l'absorption de la lumière infrarouge par les molécules de dioxyde de carbone. Cette méthode fournit des mesures précises et sensibles, mais peut nécessiter une instrumentation et un étalonnage complexes. En revanche, les capteurs électrochimiques offrent une approche plus simple et plus rentable pour la détection du dioxyde de carbone.

En outre, des capteurs à électrolyte solide fonctionnant à haute température ont également été largement utilisés pour mesurer le dioxyde de carbone dissous. Ces capteurs ont été utilisés dans une large variété d'industries, y compris le contrôle environnemental, la biotechnologie, la biologie, la médecine et l'industrie alimentaire. Au-delà des capteurs électrochimiques traditionnels, d'autres méthodes telles que la spectroscopie infrarouge non dispersive, les effets photo-acoustiques et la conductivité thermique sont également explorées pour détecter le dioxyde de carbone dissous.

Les capteurs électrochimiques à électrolytes liquides sont basés sur le principe de Severinghaus, qui consiste à mesurer indirectement la pression partielle de dioxyde de carbone en contrôlant les valeurs de pH d'un électrolyte interne. Il convient toutefois de noter que si les capteurs électrochimiques de type Severinghaus sont peu coûteux et relativement simples à utiliser, ils reposent sur une mesure indirecte de la pression partielle du dioxyde de carbone et peuvent être influencés par d'autres gaz dissous ayant un comportement similaire. Cependant, de par leur facilité d'intégration et leur faible coût, ces capteurs offrent une solution pratique pour suivre en continu des concentrations de dioxyde de carbone dans diverses applications.

Le principe dit de Severinghaus **[1]** i.e. le principe de fonctionnement d'un capteur de dioxyde de carbone électrochimique repose sur un capteur de pH immergé au sein d'une chambre contenant un électrolyte en phase liquide recouvert d'une membrane perméable et sélective au CO₂. Après le passage au travers de la membrane perméable au CO₂, le gaz est dissous dans l'électrolyte aqueux. La concentration dans l'électrolyte est alors corrélée à celle dans le milieu d'intérêt.

En effet, la dissolution du CO₂ dans la solution électrolytique entraîne la formation d'ions bicarbonate (HCO₃⁻) et par conséquent une variation de pH par le biais de la cascade d'équilibre comme décrite par les équations suivantes :

*CO*_{2 (*gaz*)} ↔ *CO*_{2 (*dissous*)} *+ H*₂*O*

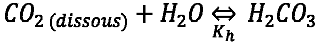

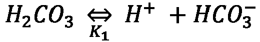

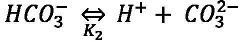

où Kₕ est la constante d'Henry, et K₁ et K₂ sont les première et seconde constantes de dissociation de l'acide carbonique (H₂CO₃). En prenant en considération la dissociation de l'eau et l'équilibre des charges, la relation entre les ions H⁺ et la pression partielle en CO₂ est déduite comme suit :

[*H*⁺]³ + [*NaHCO*₃][*H*⁺]² *-* (*K*₁*K_{H}pCO*₂ *+ K_{w}*)[*H*⁺] *-* 2*K*₁*K*₂*KₙpCO*₂ *=* 0

où [NaHCO₃] est la concentration en bicarbonate de sodium dans l'électrolyte interne et K_{w} la constante de dissociation de l'eau.

La variation du pH en fonction de la concentration en CO₂ peut ainsi être décrite par : $pH = pKa + log \left(\frac{\left[{HCO}_{3}^{-}\right]}{α {pCO}_{2}}\right)$ avec pKa, la constante de dissociation de H₂CO₃ dans l'eau dont la valeur est connue. [HCO₃⁻] est la concentration en bicarbonate fixée dans l'électrolyte. La constante de dissolution du CO₂ dans le milieu est exprimée par α qui est l'inverse de la constante de Henry du CO₂.

Cette modification du pH peut être évaluée en temps réel par une mesure de potentiel entre l'électrode sensible au pH et une électrode de référence immergée elle aussi dans l'électrolyte. Il convient toutefois de préciser que la mesure du CO₂ est obtenue de manière indirecte au travers d'un capteur pH.

Les capteurs de type Severinghaus conventionnels présentent certaines limitations qui doivent être prises en compte. L'une des caractéristiques principales de ces capteurs est l'utilisation d'une électrode interne en verre. Cependant, cette complexité de fabrication engendre des coûts élevés et une production relativement laborieuse. De plus, en raison de leur nature fragile, les électrodes en verre sont sujettes à la casse, ce qui peut nécessiter des remplacements fréquents, impacter la fiabilité des mesures et engendrer une contamination du milieu étudié. L'entretien régulier requis pour maintenir la performance optimale de ces capteurs peut également représenter une contrainte, particulièrement dans des environnements exigeants.

Une autre limitation majeure de ces capteurs conventionnels est liée à leur taille. En raison de leur conception, ces capteurs peuvent être relativement volumineux, ce qui peut limiter leur applicabilité dans des situations où l'espace est restreint, lorsque des mesures discrètes sont nécessaires, ou encore dans le contexte de dispositif multi-capteur. De plus, la présence d'une électrode en verre et les composants associés ajoutent à la taille globale de l'appareil, ce qui peut être contraignant dans des contextes où la miniaturisation est essentielle.

Les capteurs électrochimiques miniaturisés de type Severinghaus visent à résoudre certaines des limitations inhérentes aux capteurs conventionnels. Cependant, même dans cette catégorie, plusieurs défis subsistent. Ainsi, en ce qui concerne la première « couche », celle de la mesure du pH, toute lacune de cette dernière peut compromettre la précision globale du capteur, soulignant ainsi l'importance de la sélection et de l'optimisation minutieuses de cette composante. La nature de l'électrolyte interne est également un point crucial à considérer en termes de prix, de stabilité au fil du temps et de stockage à long terme. Enfin, la membrane perméable qui isole le capteur du milieu étudié joue un rôle crucial dans les performances globales du capteur puisqu'elle est en contact direct avec le milieu mesuré, ce qui rend crucial le choix d'un matériau approprié en fonction de l'application spécifique.

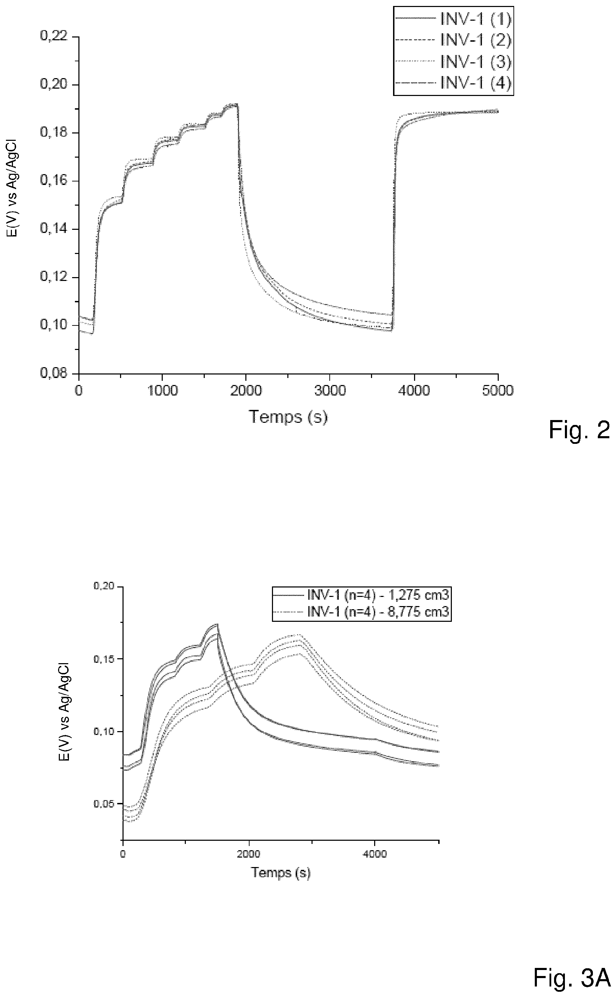

Kojima *et al,* 2005 [2] propose un capteur miniature pour surveiller la pression partielle en O₂ (*p*O₂) et en CO₂ (*p*CO₂) ainsi que le pH (Figure 2). Son support est en verre, les zones actives du capteur sont délimitées et une couche isolante en polyimide est utilisée. Les parois des chambres de mesure sont en SU-8 (polymère photosensible) et surmontées par une membrane perméable au gaz pour les zones destinées à la surveillance de la *p*CO₂ et du pH.

L'optimisation des capteurs miniaturisés de type Severinghaus requiert une approche équilibrée et minutieuse dans le choix et la combinaison de ces différentes couches. La performance, la facilité d'intégration industrielle et le coût sont étroitement liés à la synergie entre ces éléments.

Les inventeurs se sont donc fixé pour but de proposer un procédé rapide, facile à mettre en œuvre et permettant de préparer un capteur miniaturisé, efficace et fiable pour la détection et la mesure du dioxyde de carbone notamment du dioxyde de carbone dissous.

### EXPOSÉ DE L'INVENTION

La solution proposée par les inventeurs est un procédé de fabrication simple, rapide et facilement industrialisable selon la revendication indépendante 1 permettant d'obtenir un capteur de dioxyde de carbone de type Severinghaus avec une architecture spécifique selon la revendication indépendante 11, ledit capteur permettant la détection er la mesure du dioxyde de carbone et notamment du dioxyde de carbone dissous selon la revendication indépendante 13.

Le procédé de préparation d'un capteur de dioxyde de carbone selon l'invention passe donc par un choix optimal et la réalisation de différentes étapes afin d'obtenir les différentes parties constitutives du capteur que sont (i) la couche sensible au pH, (ii) le matériau de structure de la cavité interne, (iii) l'électrolyte interne et (iv) la membrane perméable.

L'architecture spécifique ainsi obtenue a démontré la capacité de développer un capteur miniaturisé de type Severinghaus présentant, comme illustré dans la partie expérimentale ci-après, tout ou partie des propriétés suivantes :
- conservant d'excellentes performances particulièrement en terme de temps de réponses et ce, même avec de faibles concentrations de CO₂ dissous ;
- pleinement intégrable dans un système multi-capteur ;
- permettant une fabrication simple et peu couteuse grâce à sa compatibilité avec des procédés de fabrication collective tels que la sérigraphie permettant d'obtenir des capteurs reproductibles à faible prix ;
- offrant une forte polyvalence en terme de support, de géométrie, ou encore de configuration ;
- pouvant, lorsque l'électrolyte interne se présente sous forme d'un hydrogel, être stocké à l'air libre et étant de fait prêt à l'emploi facilitant ainsi significativement la manipulation ; et
- ne nécessitant aucune maintenance.

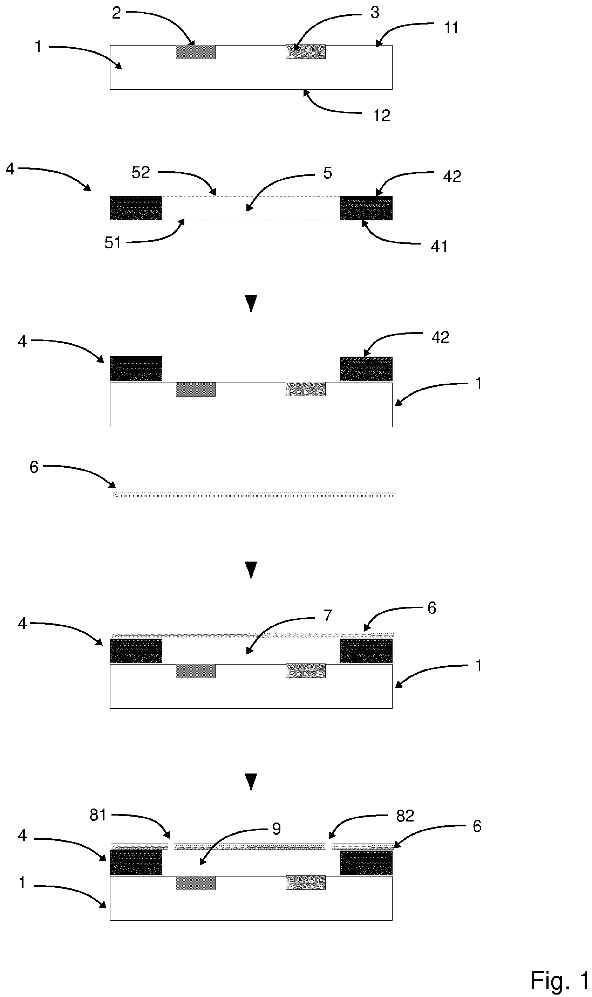

Ainsi, la présente invention concerne un procédé pour préparer un capteur de dioxyde de carbone dont les étapes sont schématisées à la Figure 1. Ce procédé comprend les étapes suivantes :
a) fournir un support 1 en un matériau isolant présentant une première face 11 et une seconde face 12 opposée à la première face, la première face présentant au moins une zone comprenant une électrode sensible au pH 2 et une électrode de référence 3 ;
b) fournir un adhésif double face 4 présentant une première couche adhésive 41 à base d'acrylique, une seconde couche adhésive 42 à base de silicone opposée à la première couche adhésive et, dans son épaisseur, un évidement traversant 5 présentant une première extrémité ouverte 51 et une seconde extrémité ouverte 52 opposée à la première extrémité ouverte ;
c) appliquer la première couche adhésive 41 de l'adhésif double face 4 sur la première face 11 du support 1 de sorte que la première extrémité ouverte 51 de l'évidement 5 de l'adhésif double face 4 vienne en regard de la zone de la première face 11 du support 1 ;
d) appliquer, sur la seconde couche adhésive 42 de l'adhésif double face 4 et sur la seconde extrémité ouverte 52 de l'évidement 5 de l'adhésif double face 4, une membrane perméable au dioxyde de carbone 6 à base de silicone moyennant quoi une chambre de mesure 7 est obtenue, puis
e) remplir la chambre de mesure 7 ainsi obtenue avec un électrolyte 9 comprenant des ions bicarbonate (HCO₃⁻) moyennant quoi l'électrode sensible au pH 2 et l'électrode de référence 3 sont en contact avec l'électrolyte.

Le support (ou substrat) du capteur de CO₂ préparé par le procédé selon la présente invention peut être n'importe quel substrat en un matériau isolant et notamment n'importe quel substrat en un matériau isolant typiquement utilisé dans les capteurs de CO₂ de type Severinghaus. Il peut également être sensiblement plan ou, au contraire, concave ou convexe et d'une forme quelconque telle qu'une forme carrée, rectangulaire, ronde ou ovale.

Avantageusement, le matériau isolant i.e. non conducteur de l'électricité du support mis en œuvre dans le procédé selon l'invention est choisi dans le groupe constitué par les oxydes non conducteurs, les polymères non conducteurs, les matériaux isolants amorphes, les matériaux isolants cristallins et l'une quelconque de leurs combinaisons.

En particulier, le matériau isolant du support mis en œuvre dans le procédé selon l'invention est choisi dans le groupe constitué par le dioxyde de silicium (SiO₂), l'oxyde d'aluminium (Al₂O₃), l'oxyde de magnésium (MgO), un verre contenant généralement des silicates, un verre de silice, une céramique, du diamant, un polysulfure de phénylène, un polyéthylèneimine, un polytétrafluoroéthylène, un polyimide, un polyéthylène, un polypropylène, un polystyrène, un polycarbonate, un polyméthyl méthacrylate, un polysulfone, un polyetherimide, un polyether ether cétone, le parylène N^{™}, le Nuflon^{™}, un silicone, une résine époxyde et l'une quelconque de leurs combinaisons.

Le support mis en œuvre dans l'invention peut être souple ou rigide. Dans un mode de réalisation particulier, ce support est un substrat typiquement utilisé pour les circuits imprimés (ou « PCB » pour « Printed Circuit Board »). Un tel type de substrat est généralement constitué de couches isolantes en résine époxyde renforcée par une trame de fibres de verre ou de papier.

Le support mis en œuvre dans le cadre du procédé selon l'invention peut présenter une seule zone comprenant une électrode sensible au pH et une électrode de référence. En variante, le support mis en œuvre dans le cadre du procédé selon l'invention peut présenter plusieurs zones, identiques ou différentes et notamment de taille et forme identiques ou différentes, chaque zone comprenant une électrode sensible au pH et une électrode de référence.

La préparation du support mis en œuvre dans le cadre du procédé selon l'invention avec au moins une zone comprenant une électrode sensible au pH et une électrode de référence est un procédé classique dans la préparation des capteurs de CO₂ de type Severinghaus. A titre d'exemples de techniques classiquement utilisées pour cette préparation, on peut citer une fabrication collective par sérigraphie ou par procédé silicium ou encore des techniques d'impression jet d'encre.

L'électrode sensible au pH que présente le support mis en œuvre dans le cadre du procédé selon l'invention présente (i) au moins une couche conductrice de l'électricité disposée sur le support en un matériau isolant i.e. la face inférieure de la couche conductrice est en contact direct avec la première face du support telle que précédemment définie et (ii) au moins une couche sensible au pH comprenant des éléments sensibles au pH, directement ou indirectement, disposée sur cette couche conductrice de l'électricité.

La couche conductrice de l'électrode sensible au pH que présente le support mis en œuvre dans le cadre du procédé selon l'invention permet de relier électriquement cette électrode à la partie électronique de mesure. Elle peut être en tout matériau conducteur de l'électricité et notamment n'importe quel matériau conducteur de l'électricité typiquement utilisé dans les capteurs de CO₂ de type Severinghaus.

Avantageusement, la couche conductrice est en un matériau conducteur de l'électricité choisi dans le groupe constitué par les métaux tels que les métaux nobles, les carbones et les polymères ou copolymères conducteurs de l'électricité.

Plus particulièrement, la couche conductrice de l'électrode sensible au pH que présente le support mis en œuvre dans le cadre du procédé selon l'invention est en un métal choisi dans le groupe constitué par l'or, le cuivre, l'inox, l'argent, le nickel, l'aluminium, le titane, le platine, le palladium, le ruthénium, l'iridium, le molybdène ou un de leurs alliages.

Lorsque la couche sensible au pH est indirectement disposée sur la couche conductrice de l'électricité, une couche comprenant un transducteur électrochimique est disposée entre ces deux couches. En variante, lorsque la couche sensible au pH est directement disposée sur la couche conductrice de l'électricité, la couche sensible au pH comprend non seulement des éléments sensibles au pH mais aussi un transducteur électrochimique.

Le transducteur électrochimique dans l'électrode sensible au pH que présente le support mis en œuvre dans le cadre du procédé selon l'invention est l'élément de cette électrode dont la fonction est de retranscrire la reconnaissance chimique de la couche sensible au pH en un signal électrique i.e. un signal physique mesurable, sensible et facilement exploitable.

Tout transducteur électrochimique classiquement utilisé dans les capteurs de CO₂ de type Severinghaus est utilisable dans le cadre de la présente invention. Typiquement, le transducteur électrochimique est choisi parmi les nanomatériaux à base de carbone tels que, par exemple, des nanotubes de carbone mono-paroi ou multi-parois, des nanofils de carbone et du graphène ; les encres carbone telles que, par exemple, l'encre à base de graphite C10903P14 (Gwent Electronic. Materials Ltd, Montypool, Royaume-Uni) ou l'encre à base de carbone BQ242 (DuPont, Bristol, Royaume-Uni) ; et les polymères semiconducteurs tels que, par exemple, du poly(3,4-éthylènedioxythiophène) couplé au poly(styrène sulfonate) de sodium (PEDOT:PSS) ou du poly(3-octylthiophene-2,5-diyl).

L'homme du métier connait différents éléments sensibles au pH utilisables pour l'électrode sensible au pH mise en œuvre dans l'invention. Avantageusement, les éléments sensibles au pH de l'électrode sensible au pH que présente le support mis en œuvre dans le cadre du procédé selon l'invention sont en un matériau choisi parmi les oxydes de métaux de transition.

En particulier, les éléments sensibles au pH de l'électrode sensible au pH que présente le support mis en œuvre dans le cadre du procédé selon l'invention se trouvent sous forme nanométrique et notamment sous forme de poudre, de grains, de paillettes, de particules ou d'un de leurs mélanges et sont en un matériau choisi dans le groupe constitué par l'oxyde de titane (TiO₂), l'oxyde de ruthénium (RuO₂), l'oxyde de palladium (PdO₂), l'oxyde de platine (PtO₂), l'oxyde d'iridium (IrO₂), l'oxyde de tungstène (WO₂) et leurs mélanges.

Plus particulièrement, les éléments sensibles au pH de l'électrode sensible au pH que présente le support mis en œuvre dans le cadre du procédé selon l'invention sont en oxyde d'iridium (IrO₂).

Le support du capteur de CO₂ préparé par le procédé selon l'invention comprend, en plus, une électrode de référence. Toute électrode de référence ou électrode de pseudo-référence classiquement utilisée dans les capteurs de CO₂ de type Severinghaus est utilisable dans le cadre de la présente invention. Avantageusement, l'électrode de référence que présente le support mis en œuvre dans le cadre du procédé selon l'invention est une électrode de pseudo-référence au chlorure d'argent (AgCl/Ag).

Dans le procédé de préparation selon l'invention, un adhésif double face est utilisé. Les trois éléments essentiels de cet adhésif double face sont une première couche adhésive à base d'acrylique, une seconde couche adhésive à base de silicone et un évidement traversant. L'adhésif double face mis en œuvre dans le cadre du procédé selon l'invention présente des couches adhésives de compositions chimiques différentes : il s'agit donc d'un adhésif double face asymétrique.

La première couche adhésive à base d'acrylique de cet adhésif double face est destinée à être appliquée sur la première face du support telle que précédemment décrite ce qui permet de réaliser une adhésion forte et un scellement efficace au niveau du support. De même, la seconde couche adhésive à base de silicone a une très bonne affinité avec les silicones et notamment le silicone de la membrane perméable au CO₂ destinée à être appliquée sur cette seconde couche adhésive.

Enfin, l'évidement traversant que présente l'adhésif double face mis en œuvre est destiné à former les parois de la chambre de mesure ou cavité du capteur de CO₂ dans laquelle se trouvent l'électrode sensible au pH, l'électrode de référence et l'électrolyte. Typiquement, la forme de la première extrémité ouverte de cet évidement est sensiblement identique à la forme de la zone de la première face du support en un matériau isolant telle que précédemment définie. Avantageusement, cet évidement présente une forme sensiblement parallélépipédique. L'usinage d'un tel évidement est un travail de routine pour l'homme du métier soit de manière manuelle en utilisant un emporte-pièce ou un cutter, soit en fabrication collective par découpe mécanique ou laser.

Typiquement, l'adhésif double face mis en œuvre dans le cadre de la présente invention comprend, entre la première couche adhésive à base d'acrylique et la seconde couche adhésive à base de silicone, un substrat polymérique et notamment un substrat en polyester. Avantageusement, l'adhésif double face mis en œuvre dans l'invention présente une épaisseur comprise entre 50 µm et 585 µm et notamment une épaisseur de l'ordre de 85 µm (i.e. 85 µm ± 10 µm).

Tout adhésif double face asymétrique notamment commercial qui présente les caractéristiques ci-dessus est utilisable dans la cadre du procédé de préparation selon l'invention. A titre d'exemple particulier, on peut citer l'adhésif double face de référence 5302A et commercialisé par la société NITTO DENKO.

Une fois l'adhésif double face placé sur la première face du support, on place, sur la seconde couche adhésive à base de silicone de l'adhésif double face et sur la seconde extrémité ouverte de l'évidement de l'adhésif double face, une membrane perméable au CO₂ à base de silicone. Typiquement, cette membrane perméable au CO₂ est un film de silicone réticulé.

Avantageusement, la membrane perméable au CO₂ mise en œuvre dans le procédé selon l'invention présente une épaisseur comprise entre 20 µm et 400 µm et notamment une épaisseur de l'ordre de 50 µm (i.e. 50 µm ± 10 µm).

Toute membrane perméable au CO₂ présentant les caractéristiques ci-dessus est utilisable dans le cadre du procédé de préparation selon l'invention. Toutefois, des membranes perméables fabriquées à partir d'une formulation commerciale en deux parties (MED-6010, Nusil) enduite par centrifugation (ou en anglais « spin-coating ») sur un wafer en silicium afin d'obtenir une membrane la plus fine possible puis suivi d'un transfert sur l'adhésif double face ont montré des problèmes de reproductibilité dus principalement à des difficultés à transférer les silicones engendrant ainsi des aspérités et/ou des fissures sur la membrane une fois transférée. Dans l'objectif de résoudre ce problème et de simplifier une future industrialisation du procédé, un procédé optimal a été déterminée avec des films de silicones commerciaux. L'intégration de ces silicones dans le procédé de fabrication des capteurs de dioxyde de carbone a montré une excellente reproductibilité des transferts tout en facilitant la fabrication.

Ainsi, la membrane perméable au CO₂ mise en œuvre dans le procédé selon l'invention est une membrane commerciale et notamment un film de silicone réticulé de référence SILPURAN^{®} Film 2030 commercialisé par la société Wacker Chemie AG.

Les parois latérales de la chambre de mesure ou cavité du capteur de CO₂ préparé par le procédé selon la présente invention correspondent aux bords latéraux de l'évidement de l'adhésif double face. La partie inférieure de cette chambre de mesure correspond au support en un matériau isolant au niveau de la zone telle que précédemment définie et la partie supérieure de cette chambre de mesure correspond à la membrane perméable au CO₂ recouvrant la seconde extrémité ouverte de l'évidement de l'adhésif double face.

Le procédé selon la présente invention permet d'obtenir des chambres de mesure dont le volume est faible ce qui permet d'obtenir des capteurs de CO₂ très performants (cf partie expérimentale ci-après). La chambre de mesure obtenue lors du procédé de préparation selon l'invention présente un volume compris entre 1 cm³ et 12 cm³, notamment entre 1,1 cm³ et 9 cm³ et, en particulier, de l'ordre de 1,275 cm³ (i.e. 1,275 cm³ ± 0,05 cm³). A titre d'exemples particuliers, la chambre de mesure obtenue lors du procédé de préparation selon l'invention peut présenter un volume de l'ordre de 1,275 cm³, de l'ordre de 5,025 cm³ (i.e. 5,025 cm³ ± 0,05 cm³) et de l'ordre de 8,775 cm³ (i.e. 8,775 cm³ ± 0,05 cm³).

L'étape e) du procédé de préparation selon l'invention consiste à remplir la chambre de mesure avec un électrolyte. Ce remplissage est réalisé au moyen d'au moins deux canaux fluidiques **81, 82** débouchant dans la chambre de mesure et adaptés pour amener, dans la chambre de mesure, un électrolyte ou un précurseur de ce dernier et pour extraire l'air contenu dans la chambre de mesure. Ces canaux fluidiques sont typiquement des microcanaux fluidiques, percés au niveau de la membrane perméable au CO₂ et/ou usinés au niveau de l'adhésif double face, notamment au niveau du substrat polymérique et, en particulier, au niveau du substrat en polyester que comprend cet adhésif double face. Dans un mode de réalisation particulier, la membrane perméable est percée notamment à l'aide d'une aiguille associée à une seringue pour l'injection de l'électrolyte ou du précurseur de ce dernier. Le silicone de la membrane perméable et notamment de la membrane perméable commerciale telle que précédemment définie est « auto-régénérant ». Toutefois, pour s'assurer que la chambre de mesure ou cavité interne soit bien hermétique, un second adhésif ou une seconde membrane perméable peut être ajouté(e) par-dessus l'ensemble.

Dans un premier mode de réalisation, l'électrolyte mis en œuvre dans le procédé de préparation selon la présente invention est un électrolyte liquide. Le ou les canaux fluidiques tels que précédemment définis permettent d'amener un tel électrolyte liquide dans la chambre de mesure.

Dans un second mode de réalisation, l'électrolyte mis en œuvre dans le procédé de préparation selon la présente invention est un électrolyte se présentant sous forme d'un hydrogel. Un électrolyte interne sous la forme d'un hydrogel permet de limiter voire de supprimer l'évaporation lors des phases de stockage et d'utilisation du capteur de CO₂, ainsi que de simplifier la fabrication.

Dans ce second mode de réalisation, pour éviter tout bouchage du ou des canaux fluidiques tels que précédemment définis, c'est une solution comprenant les composés précurseurs de cet hydrogel qui sera amenée, via ce ou ces canaux fluidiques, dans la chambre de mesure.

Typiquement, un hydrogel désigne un matériau formé d'au moins deux constituants : une solution, également appelée solution gélifiée, qui est un liquide « piégé » par un second composé qui forme un réseau tridimensionnel dans l'ensemble de la solution, la solution d'un hydrogel étant de l'eau ou une solution aqueuse i.e. une solution dont le solvant est de l'eau.

Dans la présente invention, tous les composés précurseurs généralement employés pour préparer un hydrogel peuvent être utilisés. Avantageusement, ces composés précurseurs sont choisis dans le groupe constitué par du chitosane, du xanthane, de la carraghénine, du dextrane, de l'agar, de l'alginate, de la gélatine, du collagène, de la fibrine, du polyéthylène glycol, de l'acide hyaluronique, leurs dérivés (méth)acrylés et leurs mélanges.

Dans un mode de réalisation particulier, l'électrolyte se présentant sous forme d'un hydrogel mis en œuvre dans l'invention est un hydrogel obtenu à partir de précurseurs choisis dans le groupe constitué par l'agar, la gélatine, du chitosane et du dextrane méthacrylé. Dans ce mode de réalisation particulier, la solution comprenant les composés précurseurs d'un tel hydrogel comprend typiquement 1% en masse d'agar, 25% en masse de gélatine, 10% en masse de chitosane ou 5% en masse de dextrane méthacrylé.

Dans un mode de réalisation plus particulier encore, l'électrolyte se présentant sous forme d'un hydrogel mis en œuvre dans l'invention est un hydrogel d'agar. Dans ce mode de réalisation plus particulier encore, la solution comprenant les composés précurseurs d'un tel hydrogel comprend 1% en masse d'agar.

Que l'électrolyte mis en œuvre dans le cadre de la présente invention soit sous forme liquide ou sous forme d'un hydrogel, il comprend des ions bicarbonate en une quantité comprise entre 1 mM et 20 mM, notamment entre 3 mM et 10 mM et, en particulier, de l'ordre de 5 mM (i.e. 5 mM ± 1 mM). Cette concentration en ions bicarbonate s'entend dans l'électrolyte liquide ou dans la solution comprenant les composés précurseurs de l'hydrogel formant l'électrolyte. Les ions bicarbonate sont typiquement présents sous forme de bicarbonate de sodium (NaHCO₃).

Avantageusement, dans le cadre du procédé selon l'invention, l'électrolyte liquide et la solution comprenant les composés précurseurs de l'hydrogel formant l'électrolyte comprennent en outre un sel dans lequel l'anion est un chlorure (Cl⁻). Avantageusement, le cation de ce sel est un cation métallique alcalin tels que Li⁺, Na⁺, K⁺; un cation métallique alcalino-terreux tels que Mg²⁺, Ca²⁺; ou un cation métallique d'un métal de transition tels que Cu²⁺, Zn²⁺ et Al³⁺. Dans un mode de réalisation particulier, le sel mis en œuvre dans l'électrolyte liquide et la solution comprenant les composés précurseurs de l'hydrogel formant l'électrolyte est du KCl. Plus particulièrement, ce sel est présent dans l'électrolyte liquide et la solution comprenant les composés précurseurs de l'hydrogel formant l'électrolyte en une quantité comprise entre 25 mM et 250 mM, notamment entre 50 mM et 150 mM et, en particulier, de l'ordre de 100 mM (i.e. 100 mM ± 10 mM).

Enfin, la solution comprenant les composés précurseurs de l'hydrogel formant l'électrolyte mise en œuvre dans le cadre du procédé de préparation selon l'invention peut comprendre au moins un élément nécessaire à la formation d'un hydrogel à partir de ces composés précurseurs. Un tel élément peut être, par exemple, un agent de réticulation comme l'éther diglycidylique de polyéthylène glycol (ou PEGDE) ou un photoinitiateur comme le phényl-2,4,6-triméthylbenzoylphosphinate de lithium (ou LAP).

A noter qu'il est possible, avant l'étape e) du procédé de l'invention i.e. avant de remplir la chambre de mesure du capteur avec l'électrolyte, de soumettre l'ensemble obtenu à l'issue de l'étape d), ensemble comprenant le support en un matériau isolant, l'électrode sensible au pH, l'électrode de référence, l'adhésif double face et la membrane perméable au CO₂ tels que précédemment définis i.e. les capteurs sans électrolyte interne à une étape de stérilisation notamment par autoclave ou par irradiation gamma. En effet, aucune détérioration de la structure des capteurs suite à une telle stérilisation n'a été observée.

La présente invention concerne également un capteur de CO₂ obtenu par le procédé de préparation tel que précédemment défini. Ce capteur comprend :
- un support **1** en un matériau isolant présentant une première face **11** et une seconde face **12** opposée à la première face, la première face présentant au moins une zone comprenant une électrode sensible au pH **2** et une électrode de référence **3 ;**
- un adhésif double face **4** présentant une première couche adhésive **41** à base d'acrylique, une seconde couche adhésive **42** à base de silicone opposée à la première couche adhésive et, dans son épaisseur, un évidement traversant **5** présentant une première extrémité ouverte **51 et** une seconde extrémité ouverte **52** opposée à la première extrémité ouverte ;
   la première couche adhésive **41** de l'adhésif double face **4** étant en contact avec (i.e. appliquée sur) la première face **11** du support **1** de sorte que la première extrémité ouverte **51** de l'évidement **5** de l'adhésif double face **4** soit en regard de la zone de la première face **11 du** support **1 ;**
- une membrane perméable au dioxyde de carbone **6** à base de silicone en contact avec (i.e. appliquée sur) la seconde couche adhésive **42** de l'adhésif double face **4** et en contact avec (i.e. appliquée sur) la seconde extrémité ouverte **52** de l'évidement **5** de l'adhésif double face **4 ;**
- une chambre de mesure **7** dont les parois latérales correspondent aux bords latéraux de l'évidement **5** de l'adhésif double face **4,** dont la partie inférieure correspond au support **1** en un matériau isolant au niveau de la zone telle que précédemment définie et dont la partie supérieure correspond à la membrane perméable au CO₂ **6** recouvrant la seconde extrémité ouverte **52** de l'évidement **5** de l'adhésif double face **4 ;**
ladite chambre de mesure étant remplie avec un électrolyte **9** comprenant des ions bicarbonate (HCO₃⁻).

Les différents éléments que comprend le capteur selon la présente invention sont tels que précédemment définis dans le cadre du procédé de préparation selon la présente invention.

La présente invention concerne aussi un dispositif multi-capteurs comprenant au moins un capteur de CO₂ tel que précédemment défini. Dans un tel dispositif multi-capteurs, le capteur de CO₂ peut être associé, par exemple, à un ou plusieurs capteurs choisi dans le groupe constitué par les capteurs de température, les capteurs d'humidité relative et les capteurs électrochimiques comme, par exemple, les capteurs métaboliques, les capteurs pH et les capteurs ioniques.

La présente invention concerne enfin l'utilisation d'un capteur de CO₂ tel que précédemment défini ou d'un dispositif multi-capteurs tel que précédemment défini pour mesurer (i) le CO₂ dissous dans un milieu liquide en contact avec ledit capteur ou ledit dispositif multi-capteurs comme, par exemple, de l'eau ou un fluide biologique ou (ii) le CO₂ gazeux présent dans un fluide gazeux en contact avec ledit capteur ou ledit dispositif multi-capteurs.

A titre d'exemples d'applications d'une telle utilisation, on peut citer :
- dans l'industrie chimique et notamment pharmaceutique : Par exemple, le CO₂ dissous est un paramètre critique dans les processus de production biopharmaceutique selon les directives PAT. En influençant d'autres paramètres tels que le pH extracellulaire et intracellulaire, il a un effet sur différentes voies métaboliques qui interviennent dans la croissance ou dans la formation et la qualité des produits ;
- dans l'analyse physiologique et le diagnostic médical : Par exemple, des capteurs portés à la personne pour le suivi de la pression artérielle de dioxyde de carbone (PaCO₂) de patients atteints d'insuffisance respiratoire ;
- dans l'industrie agroalimentaire : Par exemple, pour le brassage ou la fabrication du vin, dans une brasserie, le CO₂ dissous est mesuré pour surveiller les attributs de qualité afin notamment de garantir une sensation au goût identique ;
- dans le domaine industriel : Par exemple, la mesure du CO₂ dissous est un paramètre très important pour l'optimisation des procédés industriels ;
- dans l'analyse environnementale et notamment pour la qualité de l'eau : Par exemple, la mesure du CO₂ dissous est un paramètre très important pour l'évaluation de la qualité de l'eau des étangs en pisciculture et la surveillance des eaux marines et d'eau douce.

D'autres caractéristiques et avantages de la présente invention apparaîtront encore à l'homme du métier à la lecture des exemples ci-après donnés à titre illustratif et non limitatif, en référence aux figures annexées.

### BRÈVE DESCRIPTION DES DESSINS

La Figure 1 déjà présentée correspond à la schématisation du procédé de préparation d'un capteur de CO₂ selon l'invention.
La Figure 2 présente un exemple de réponses de capteurs de CO₂ dissous selon l'invention. Réponses à différentes concentrations de CO₂ dissous de 4 capteurs selon l'invention (INV-1) avec une épaisseur d'adhésif 5302A (Nitto) et une membrane perméable en Silpuran 2030 (Wacker).
La Figure 3A présente la caractérisation de l'impact du volume d'électrolyte interne sur le temps de réponse des capteurs de CO₂ dissous selon l'invention.
La Figure 3B présente les temps de réponses indiqués correspondent au T99% pour différents volumes d'électrolytes interne liquide (5 mM NaHCO₃ et 0,1 M KCl).
La Figure 4 présente les réponses de 3 capteurs de CO₂ dissous selon l'invention (INV-1) lors de changements de solutions de PBS (250 mM) ayant des concentrations en CO₂ dissous différentes. La courbe en trait pointillé correspond à la moyenne des réponses des capteurs dissous selon l'invention (INV-1) et la zone en transparence à l'écart-type standard. La courbe en trait continu correspond à la réponse d'un capteur optique commercial (CE-1) et la zone en transparence représente la plage de justesse attendue, ±10% de la valeur de référence.
La Figure 5 présente les interférences de la conductivité, du pH et de l'oxygène dissous sur 4 capteurs de CO₂ selon l'invention (INV-1) comparés à un capteur commercial (CE-1).
La Figure 6 présente la réponse de 4 capteurs de CO₂ dissous selon l'invention (INV-1) avec un électrolyte interne liquide (5 mM NaHCO₃, 100 mM KCl) en trait continu et de 4 capteurs de CO₂ dissous selon l'invention (INV-2) avec un électrolyte à base d'AGAR (1%) en trait pointillé.

### EXPOSÉ DÉTAILLÉ DE MODES DE RÉALISATION PARTICULIERS

### I. Préparation des capteurs de CO₂.

### I.1. Capteurs de CO₂ conformes à l'invention.

La preuve de concept du procédé de fabrication et de l'intégration des capteurs de dioxyde de carbone dissous de la présente invention a été réalisée avec deux exemples INV-1 et INV-2 dont le substrat est en résine époxyde renforcée par une trame de fibres de verre ou de papier (PCB) et l'électrode de référence est une électrode de pseudo-référence en Ag/AgCl. Les autres éléments constituant ces capteurs sont tels que définis dans le Tableau 1 ci-après.

**Tableau 1**

| | Electrode sensible au pH | Film formant la cavité | Electrolyte | Membrane perméable au CO₂ |
|---|---|---|---|---|
| INV-1 | IrOx | Adhésif Nitto 5302A | NaHCO3 (5 mM) | Silpuran^{®} 2030 50 µm |
| | | | KCI (100 mM) | |
| | | | Eau DI | |
| INV-2 | IrOx | Adhésif Nitto 5302A | Agar (1%) | Silpuran^{®} 2030 50 µm |
| | | | NaHCO3 (5 mM) | |
| | | | KCI (100 mM) | |
| | | | Eau DI | |

Les électrodes sensibles au pH utilisées dans les capteurs selon l'invention sont préparées comme suit :
- Etape de formulation et mélange : utilisation de l'encre commerciale BQ242 (DuPont, Bristol, Royaume-Uni) sans modification et mélange avec de la poudre d'oxyde d'iridium (IrO₂), le ratio massique en matière sèche d'IrO₂ par rapport à BQ242 étant de 6%,
- Dépôt sur une couche conductrice en or : procédé de sérigraphie,
- Séchage : 2-3 heures à 80°C.

Le procédé de préparation générique d'un capteur de CO₂ consiste, chronologiquement, en :
- fabrication d'un capteur pH basé par fabrication collective (par sérigraphie ou par procédé silicium), sur le substrat **1** en résine époxyde renforcée par une trame de fibres de verre ou de papier (PCB) avec pistes conductrices en or, de l'électrode sensible au pH **2** basé sur un oxyde métallique et de l'électrode de référence **3** en Ag/AgCl ;
- usinage et dépôt d'un adhésif double face **4** aux géométries de la cavité interne du capteur pour obtenir le volume souhaité ;
- transfert par collage de la membrane perméable au CO₂ **5** ;
- injection de l'électrolyte interne **9** ou de son précurseur à l'aide de micro canaux fluidiques **81, 82** percés au niveau de la membrane perméable au CO₂ **5** ou usinés au niveau de l'adhésif double face **4.**

### I.2. Capteur de CO₂ de l'art antérieur.

Afin de mettre en évidence la fonctionnalité et les performances des capteurs selon l'invention, un contre-exemple a été réalisé et étudié dans les mêmes conditions avec un capteur optique commercial de la marque Presens (CE-1).

### II. Caractérisation et tests.

### II.1. Méthodes.

Pour les deux exemples, les capteurs sont immergés dans des solutions, connectés à des potentiostats qui permettent de mesurer les valeurs des potentiels à circuit ouvert pour différentes solutions (changements de solution de concentration en NaHCO₃ connues ou ajouts dosés de gaz (CO₂ ou de N₂)).

### II.2. Résultats.

### Validation de principe effectuée

La Figure 2 illustre une réponse typique lors de changements de solutions avec des concentrations en NaHCO₃ différentes de 4 capteurs de CO₂ dissous (INV-1) fabriqués en simultané sur un même substrat selon le procédé tel que précédemment défini (données brutes sans traitement). On peut observer une bonne reproductibilité inter-capteurs.

### Effet du volume de l'électrolyte

Le temps de réponse des capteurs à des variations de CO₂ dissous est un critère crucial. Pour rappel, le fait d'ajouter une membrane sur le capteur va ralentir la diffusion des espèces chimiques au contact de l'électrode, augmentant donc le temps de réponse. Ce phénomène est d'autant plus accentué lorsque le volume de l'électrolyte augmente puisque la distance parcourue par l'analyte après passage de la membrane augmente également. A ceci se rajoute le fait qu'il n'y a pas de convection mécanique dans l'enceinte.

L'influence du volume d'électrolyte interne a été étudiée en faisant varier l'épaisseur des chambres internes grâce à la flexibilité de fabrication (usinage et empilement) permise par le choix des adhésifs pour les fabriquer. Ainsi, trois volumes différents de chambre interne pour des capteurs du type INV-1 ont été caractérisés, les trois volumes testés étant 1,275 cm³, 5,025 cm³ et 8,775 cm³.

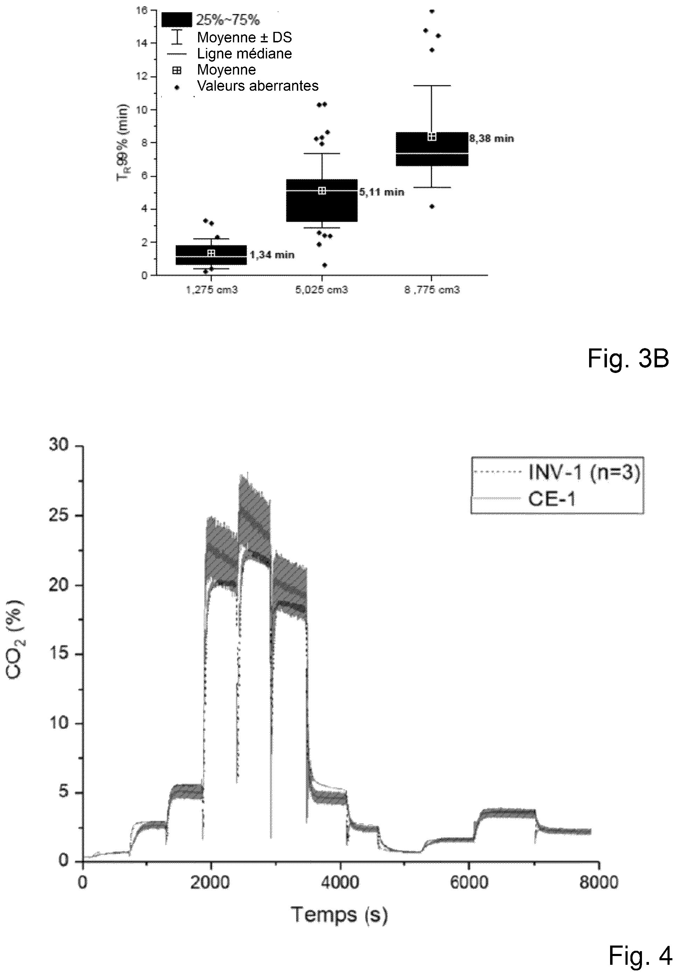

Un impact significatif a été observé, ainsi, comme décrit en amont, le temps de réponse varie avec le volume de l'électrolyte : lorsque le volume augmente, il en est de même pour le temps de réponse (Figure 3A). Les dispositifs avec les plus faibles volumes qui ont pu être fabriqués ont montré d'excellents temps de réponse moyen à 99% du signal de 1,34 min (± 0,55 SD) sur une plage de 0 à 5% de CO₂, donnée qui peut être comparée à celle indiquée par Presens de : t₉₀ < 3 min pour un changement de 2 à 5% (ou 15 mmHg - 38 mm Hg en pCO₂) (Figure 3B).

L'électrolyte interne avec le plus faible volume a permis d'obtenir des temps de réponses à 99% de l'ordre de la minute. A la date de l'invention, des temps de réponses aussi rapides à 99% du signal n'ont jamais été obtenus avec une configuration similaire de type Severinghaus.

### Caractérisation des performances

La caractérisation des performances a été réalisée par la mesure de la justesse des capteurs à partir d'un capteur commercial Presens (CE-1) et de calculer le biais relatif. Si l'on considère, comme valeur de référence, la réponse de CE-1, la justesse des capteurs développés selon l'invention peut être caractérisée par la mesure de l'éloignement absolu de leurs réponses par rapport à celle du capteur commercial.

La Figure 4 illustre les résultats obtenus. La courbe en trait pointillé correspond à la moyenne des réponses de 3 capteurs développés selon l'architecture et le procédé de fabrication de la présente invention et la zone en transparence à l'écart-type standard calculé. Cette courbe est mise en perspective avec la réponse du capteur optique commercial (CE-1, trait continu) et la zone de justesse requise de ±10% de la valeur de référence (zone en transparence). Il peut être observé deux points :
- les capteurs de CO₂ dissous selon l'invention sont justes à au moins ±10% (par rapport au capteur commercial optique) car leurs réponses recoupent la zone du capteur commercial optique;
- la fidélité des capteurs est satisfaisante car l'écart-type standard (zone en transparence) en relativement faible.

### Interférences induites par le pH, l'oxygène dissous et la température

Les interférences induites par le pH, l'oxygène dissous et la température ont ensuite été caractérisées avec différents capteurs de CO₂ dissous du type INV-1.

Après avoir réalisé une calibration par rapport au capteur commercial Presens (CE-1), les valeurs de la conductivité de la solution ont été modifiées par ajout de KCl, le pH par ajout de HCl et de NaOH, et l'oxygène dissous par bullage d'O₂ ultra pur.

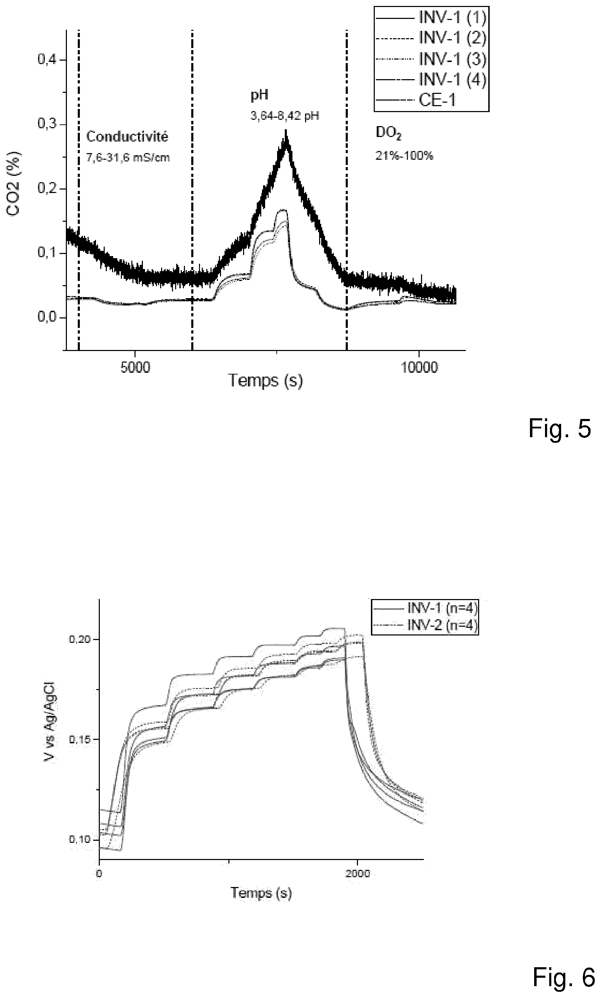

La Figure 5 illustre la réponse à ces changements de paramètres de 4 capteurs de CO₂ dissous (INV-1) en parallèle d'un capteur commercial optique (CE-1). Les résultats ont montré que les capteurs de CO₂ dissous ne sont pas affectés par des variations de conductivité du milieu de 7,6 mS/cm à 31,6 mS/cm, ni par des variations de pH de 3,64 à 8,42 unité pH, et non plus par des variations de la concentration d'oxygène dissous de 0% (argon) à 100% (O₂ ultra pur).

Enfin, ces résultats mettent en évidence une résolution significativement meilleure pour les capteurs de CO₂ dissous selon la présente invention comparés aux capteurs commerciaux de la marque Presens sur de très faibles concentrations de CO₂ dissous.

Les résultats exposés dans cette partie montrent que les capteurs de CO₂ dissous de la présente invention sont capables de fournir des mesures justes et fidèles à au moins ±10% sur une plage de mesure de 0 à 25% de CO₂ dissous. Egalement, les temps de réponse obtenus sont très satisfaisants dans des conditions variables de conductivité, d'oxygène dissous et de pH.

### Influence de l'hydrogel

Une particularité de certains des capteurs de CO₂ dissous de la présente invention est la présence d'un électrolyte interne sous la forme d'un hydrogel afin de limiter voire de supprimer l'évaporation lors des phases de stockage et d'utilisation, ainsi que de simplifier la fabrication.

Dans ce sens, plusieurs alternatives d'électrolyte sous forme d'hydrogel ont été évaluées :
- AGAR (1% en masse), 5 mM NaHCO₃, 100 mM KCl,
- Gélatine (25% en masse), 5 mM NaHCO₃, 100 mM KCl,
- Chitosane (10% en masse), éther diglycidylique de polyéthylène glycol (ou PEGDE) (40 mM), 5 mM NaHCO₃, 100 mM KCl,
- Dextran méthacrylé (5% en masse), phényl-2,4,6-triméthylbenzoylphosphinate de lithium (ou LAP) (0,5% en masse), 5 mM NaHCO₃, 100 mM KCl.

La formulation composée avec un hydrogel d'AGAR (1% en masse) a montré les meilleurs résultats en termes de temps de réponse à 99% ainsi que de facilité de préparation. Comme cela est détaillé par la Figure 6, une telle formulation montre une réponse au CO₂ dissous (sensibilité et temps de réponse à 99%) similaire aux électrolytes liquides.

De plus, une première évaluation d'un stockage à l'air ambiant de ces capteurs a été réalisée. Les capteurs ont été laissés à l'air libre pendant 24 heures, puis réhydratés par de la vapeur d'eau. En réalisant un étalonnage identique suivant cette phase de réhydratation, les capteurs de CO₂ dissous fabriqués à partir d'un électrolyte « solide » d'AGAR ont conservé une sensibilité similaire (Tableau 2 ci-après).

**Tableau 2 : Valeurs de la pente (moyenne ± SD) des régressions linéaires pour ces capteurs avec électrolyte liquide, AGAR (1% en masse) et AGAR (1% en masse) réhydraté après 24h d'évaporation à l'air ambiant (Sensibilité [Log(%CO₂)/mV])**

| Electrolyte liquide | Electrolyte Agar 1% | Electrolyte Agar 1% Séché puis réhydraté |
|---|---|---|
| 66,37 ± 0,68 (n=4) | 70,63 ± 0,32 (n=4) | 61,54 ± 6,8 (n=4) |

### Référence bibliographique

[1] Severinghaus, John W.; Bradley, A. Freeman (1958). « Electrodes for Blood pO2 and pCO2 Determination ». Journal of Applied Physiology. American Physiological Society, vol. 13, pages 515-520.
[2] Kojima et al (2005) « Microanalysis system for pO2, pCO2 and pH constructed with stacked modules », IEEE Sensors Journal, vol. 5, pages 1120-1126.

## Revendications

1. Procédé pour préparer un capteur de dioxyde de carbone comprenant les étapes suivantes :
a) fournir un support (1) en un matériau isolant présentant une première face (11) et une seconde face (12) opposée à la première face, la première face présentant au moins une zone comprenant une électrode sensible au pH (2) et une électrode de référence (3) ;
b) fournir un adhésif double face (4) présentant une première couche adhésive (41) à base d'acrylique, une seconde couche adhésive (42) à base de silicone opposée à la première couche adhésive et, dans son épaisseur, un évidement traversant (5) présentant une première extrémité ouverte (51) et une seconde extrémité ouverte (52) opposée à la première extrémité ouverte ;
c) appliquer la première couche adhésive (41) de l'adhésif double face (4) sur la première face (11) du support (1) de sorte que la première extrémité ouverte (51) de l'évidement (5 de l'adhésif double face (4) vienne en regard de la zone de la première face (11) du support (1) ;
d) appliquer, sur la seconde couche adhésive (42) de l'adhésif double face (4) et sur la seconde extrémité ouverte (52) de l'évidement (5) de l'adhésif double face (4), une membrane perméable au dioxyde de carbone (6) à base de silicone moyennant quoi une chambre de mesure (7) est obtenue ; puis
e) remplir la chambre de mesure (7) ainsi obtenue avec un électrolyte (9) comprenant des ions bicarbonate (HCO₃⁻) moyennant quoi l'électrode sensible au pH (2) et l'électrode de référence (3) sont en contact avec l'électrolyte.

2. Procédé de préparation selon la revendication 1, **caractérisé en ce que** les éléments sensibles au pH de ladite électrode sensible au pH sont en un matériau parmi les oxydes de métaux de transition et, avantageusement, sont en oxyde d'iridium (IrO₂).

3. Procédé de préparation selon la revendication 1 ou 2, **caractérisé en ce que** ladite électrode de référence que présente le support mis en œuvre dans le cadre du procédé selon l'invention est une électrode de pseudo-référence au chlorure d'argent (AgCl/Ag).

4. Procédé de préparation selon l'une quelconque des revendications 1 à 3, **caractérisé en ce que** ledit adhésif double face présente une épaisseur comprise entre 50 µm et 585 µm et notamment une épaisseur de l'ordre de 85 µm (i.e. 85 µm ± 10 µm).

5. Procédé de préparation selon l'une quelconque des revendications 1 à 4, **caractérisé en ce que** ladite membrane perméable au CO₂ présente une épaisseur comprise entre 20 µm et 400 µm et notamment une épaisseur de l'ordre de 50 µm (i.e. 50 µm ± 10 µm).

6. Procédé de préparation selon l'une quelconque des revendications 1 à 5, **caractérisé en ce que** ladite chambre de mesure présente un volume compris entre 1 cm³ et 12 cm³, notamment entre 1,1 cm³ et 9 cm³ et, en particulier, de l'ordre de 1,275 cm³ (i.e. 1,275 cm³ ± 0,05 cm³).

7. Procédé de préparation selon l'une quelconque des revendications 1 à 6, **caractérisé en ce que** le remplissage lors de ladite étape e) est réalisé au moyen d'au moins deux canaux fluidiques (81, 82) débouchant dans ladite chambre de mesure et adaptés pour amener, dans ladite chambre de mesure, un électrolyte ou un précurseur de ce dernier et pour extraire l'air contenu dans ladite chambre de mesure,
lesdits canaux fluidiques étant percés au niveau de la membrane perméable au CO₂ et/ou usinés au niveau de l'adhésif double face.

8. Procédé de préparation selon l'une quelconque des revendications 1 à 7, **caractérisé en ce que** ledit électrolyte est un électrolyte liquide.

9. Procédé de préparation selon l'une quelconque des revendications 1 à 7, **caractérisé en ce que** ledit électrolyte est un électrolyte se présentant sous forme d'un hydrogel et, avantageusement d'un hydrogel d'agar.

10. Procédé de préparation selon l'une quelconque des revendications 1 à 9, **caractérisé en ce que** lesdits ions bicarbonate sont présents, dans l'électrolyte liquide ou dans la solution comprenant les composés précurseurs de l'hydrogel formant l'électrolyte, en une quantité comprise entre 1 mM et 20 mM, notamment entre 3 mM et 10 mM et, en particulier, de l'ordre de 5 mM (i.e. 5 mM ± 1 mM).

11. Capteur de CO₂ obtenu par le procédé de préparation selon l'une quelconque des revendications 1 à 10, comprenant
- un support **1** en un matériau isolant présentant une première face **11** et une seconde face **12** opposée à la première face, la première face présentant au moins une zone comprenant une électrode sensible au pH **2** et une électrode de référence **3** ;
- un adhésif double face **4** présentant une première couche adhésive **41** à base d'acrylique, une seconde couche adhésive **42** à base de silicone opposée à la première couche adhésive et, dans son épaisseur, un évidement traversant **5** présentant une première extrémité ouverte **51** et une seconde extrémité ouverte **52** opposée à la première extrémité ouverte ;
la première couche adhésive **41** de l'adhésif double face **4** étant en contact la première face 11 du support **1** de sorte que la première extrémité ouverte **51** de l'évidement **5** de l'adhésif double face **4** soit en regard de la zone de la première face **11** du support **1;**
- une membrane perméable au dioxyde de carbone **6** à base de silicone en contact avec la seconde couche adhésive **42** de l'adhésif double face **4** et avec la seconde extrémité ouverte **52** de l'évidement **5** de l'adhésif double face **4** ;
- une chambre de mesure **7** dont les parois latérales correspondent aux bords latéraux de l'évidement **5** de l'adhésif double face **4,** dont la partie inférieure correspond au support **1** en un matériau isolant au niveau de la zone telle que précédemment définie et dont la partie supérieure correspond à la membrane perméable au CO₂ **6** recouvrant la seconde extrémité ouverte **52** de l'évidement 5 de l'adhésif double face **4 ;**
ladite chambre de mesure étant remplie avec un électrolyte **9** comprenant des ions bicarbonate (HCO₃⁻).

12. Dispositif multi-capteurs comprenant au moins un capteur de CO₂ selon la revendication **11.**

13. Utilisation d'un capteur de CO₂ selon la revendication 11 ou d'un dispositif multi-capteurs selon la revendication 12 pour mesurer (i) le CO₂ dissous dans un milieu liquide en contact avec ledit capteur ou ledit dispositif multi-capteurs ou (ii) le CO₂ gazeux présent dans un fluide gazeux en contact avec ledit capteur ou ledit dispositif multi-capteurs.

## Patentansprüche

1. Verfahren zur Vorbereitung eines Kohlendioxidsensors, umfassend die folgenden Schritte:
a) Bereitstellen eines Trägers (1) aus einem isolierenden Material, das eine erste Seite (11) und eine zweite Seite (12) gegenüber der ersten Seite aufweist, wobei die erste Seite mindestens einen Bereich aufweist, der eine pH-empfindliche Elektrode (2) und eine Referenzelektrode (3) umfasst;
b) Bereitstellen eines doppelseitigen Klebebands (4), das eine erste Klebstoffschicht (41) auf Acrylbasis, eine zweite Klebstoffschicht (42) auf Silikonbasis gegenüber der ersten Klebstoffschicht und, in seiner Dicke, eine durchgehende Aussparung (5), die ein erstes offenes Ende (51) und ein zweites offenes Ende (52) gegenüber dem ersten offenen Ende aufweist, aufweist;
c) Auftragen der ersten Klebeschicht (41) des doppelseitigen Klebebands (4) auf die erste Seite (11) des Trägers (1), sodass das erste offene Ende (51) der Aussparung (5 des doppelseitigen Klebebands (4) dem Bereich der ersten Seite (11) des Trägers (1) gegenüberliegt;
d) Auftragen, auf die zweite Klebeschicht (42) des doppelseitigen Klebebands (4) und auf das zweite offene Ende (52) der Aussparung (5) des doppelseitigen Klebebands (4), einer für Kohlendioxid durchlässigen Membran (6) auf Silikonbasis, wodurch eine Messkammer (7) erhalten wird; dann
e) Befüllen der so erhaltenen Messkammer (7) mit einem Elektrolyten (9), der Bicarbonationen (HCO₃⁻) umfasst, wodurch die pH-empfindliche Elektrode (2) und die Bezugselektrode (3) mit dem Elektrolyten in Kontakt stehen.

2. Vorbereitungsverfahren nach Anspruch 1, **dadurch gekennzeichnet, dass** die pH-empfindlichen Elemente der pH-empfindlichen Elektrode aus einem Material bestehen, das zu den Übergangsmetalloxiden gehört und vorzugsweise aus Iridiumoxid (IrO₂) bestehen.

3. Vorbereitungsverfahren nach Anspruch 1 oder 2, **dadurch gekennzeichnet, dass** die Referenzelektrode, die der im Rahmen des erfindungsgemäßen Verfahrens eingesetzte Träger aufweist, eine Silberchlorid-Pseudo-Referenzelektrode (AgCl/Ag) ist.

4. Vorbereitungsverfahren nach einem der Ansprüche 1 bis 3, **dadurch gekennzeichnet, dass** das doppelseitige Klebeband eine Dicke zwischen 50 µm und 585 µm und insbesondere eine Dicke von etwa 85 µm (d. h. 85 µm ± 10 µm) aufweist.

5. Vorbereitungsverfahren nach einem der Ansprüche 1 bis 4, **dadurch gekennzeichnet, dass** die CO₂-durchlässige Membran eine Dicke zwischen 20 µm und 400 µm und insbesondere eine Dicke von etwa 50 µm (d. h. 50 µm ± 10 µm) aufweist.

6. Vorbereitungsverfahren nach einem der Ansprüche 1 bis 5, **dadurch gekennzeichnet, dass** die Messkammer ein Volumen zwischen 1 cm³ und 12 cm³ aufweist, insbesondere zwischen 1,1 cm³ und 9 cm³ und insbesondere von etwa 1,275 cm³ (d. h. 1,275 cm³ ± 0,05 cm³).

7. Vorbereitungsverfahren nach einem der Ansprüche 1 bis 6, **dadurch gekennzeichnet, dass** das Befüllen in Schritt e) mittels mindestens zweier fluidischer Kanäle (81, 82) erfolgt, die in die Messkammer münden und dazu ausgelegt sind, einen Elektrolyten oder einen Vorläufer davon in die Messkammer zu führen und um die in der Messkammer enthaltene Luft zu extrahieren,
wobei die fluidischen Kanäle im Bereich der CO₂-durchlässigen Membran durchbrochen und/oder im Bereich des doppelseitigen Klebebands bearbeitet sind.

8. Vorbereitungsverfahren nach einem der Ansprüche 1 bis 7, **dadurch gekennzeichnet, dass** der Elektrolyt ein flüssiger Elektrolyt ist.

9. Vorbereitungsverfahren nach einem der Ansprüche 1 bis 7, **dadurch gekennzeichnet, dass** der Elektrolyt ein Elektrolyt in Form eines Hydrogels und vorzugsweise eines Agarhydrogels ist.

10. Vorbereitungsverfahren nach einem der Ansprüche 1 bis 9, **dadurch gekennzeichnet, dass** die Bicarbonationen in dem flüssigen Elektrolyten oder in der Lösung, die die Vorläuferverbindungen des elektrolytbildenden Hydrogels umfasst, in einer Menge zwischen 1 mM und 20 mM, insbesondere zwischen 3 mM und 10 mM und insbesondere von etwa 5 mM (d. h. 5 mM ± 1 mM) vorhanden sind.

11. CO2-Sensor, der durch das Vorbereitungsverfahren nach einem der Ansprüche 1 bis 10 erhalten wurde, umfassend
- einen Träger **1** aus einem isolierenden Material, der eine erste Seite **11** und eine zweite Seite **12** gegenüber der ersten Seite aufweist, wobei die erste Seite mindestens einen Bereich aufweist, der eine pH-empfindliche Elektrode **2** und eine Referenzelektrode **3** umfasst;
- ein doppelseitiges Klebeband **4,** das eine erste Klebstoffschicht **41** auf Acrylbasis, eine zweiten Klebstoffschicht **42** auf Silikonbasis gegenüber der ersten Klebstoffschicht und, in seiner Dicke, eine durchgehenden Aussparung **5,** die ein erstes offenes Ende **51** und ein zweites offenes Ende **52** gegenüber dem ersten offenen Ende aufweist, aufweist;
wobei die erste Klebeschicht **41** des doppelseitigen Klebebands **4** die erste Seite **11** des Trägers **1** berührt, sodass das erste offene Ende **51** der Aussparung **5** des doppelseitigen Klebebands **4** dem Bereich der ersten Seite **11** des Trägers **1** zugewandt ist;
- eine für Kohlendioxid durchlässige Membran **6** auf Silikonbasis, die in Kontakt mit der zweiten Klebeschicht **42** des doppelseitigen Klebebands **4** und mit dem zweiten offenen Ende **52** der Aussparung 5 des doppelseitigen Klebebands **4** steht;
- eine Messkammer **7,** deren Seitenwände den seitlichen Rändern der Aussparung **5** des doppelseitigen Klebebands **4** entsprechen, deren unterer Teil dem Träger **1** aus einem Isoliermaterial auf Höhe des zuvor definierten Bereichs entspricht und dessen oberer Teil der CO₂-durchlässigen Membran **6** entspricht, die das zweite offene Ende **52** der Aussparung **5** des doppelseitigen Klebebands **4** abdeckt;
wobei die Messkammer mit einem Elektrolyten **9** gefüllt ist, der Bicarbonationen (HCO₃⁻) umfasst.

12. Multisensorvorrichtung, die mindestens einen CO₂-Sensor nach Anspruch 11 umfasst.

13. Verwendung eines CO₂-Sensors nach Anspruch 11 oder einer Multisensorvorrichtung nach Anspruch 12 zur Messung (i) des gelösten CO₂ in einem flüssigen Medium, das mit dem Sensor oder der Multisensorvorrichtung in Kontakt steht, oder (ii) des gasförmigen CO₂ in einem gasförmigen Fluid, das in Kontakt mit dem Sensor oder der Multisensorvorrichtung steht.

## Claims

1. A method for preparing a carbon dioxide sensor comprising the following steps of:
a) providing a support (1) of an insulating material having a first face (11) and a second face (12) opposite to the first face, the first face having at least one zone comprising a pH-sensitive electrode (2) and a reference electrode (3);
b) providing a double-sided adhesive (4) having a first acrylic-based adhesive layer (41), a second silicone-based adhesive layer (42) opposite to the first adhesive layer and, in its thickness, a through recess (5) having a first open end (51) and a second open end (52) opposite to the first open end;
c) applying the first adhesive layer (41) of the double-sided adhesive (4) to the first face (11) of the support (1) such that the first open end (51) of the recess (5) of the double-sided adhesive (4) faces the zone of the first face (11) of the support (1);
d) applying, to the second adhesive layer (42) of the double-sided adhesive (4) and to the second open end (52) of the recess (5) of the double-sided adhesive (4), a silicone-based carbon dioxide permeable membrane (6) whereby a measuring chamber (7) is obtained; and then
e) filling the measuring chamber (7) thus obtained with an electrolyte (9) comprising bicarbonate ions (HCO₃⁻) whereby the pH-sensitive electrode (2) and the reference electrode (3) are in contact with the electrolyte.

2. The preparation method according to claim 1, **characterised in that** the pH-sensitive elements of said pH-sensitive electrode are of a material among the oxides of transition metals and, advantageously, are of iridium oxide (IrO₂).

3. The preparation method according to claim 1 or 2, **characterised in that** said reference electrode which has the support implemented in the scope of the method according to the invention is a pseudo-reference electrode with silver chloride (AgCI/Ag).

4. The preparation method according to any one of claims 1 to 3, **characterised in that** said double-sided adhesive has a thickness between 50 µm and 585 µm and in particular a thickness of about 85 µm (i.e. 85 µm ± 10 µm).

5. The preparation method according to any one of claims 1 to 4, **characterised in that** said CO₂-permeable membrane has a thickness between 20 µm and 400 µm and in particular a thickness of about 50 µm (i.e. 50 µm ± 10 µm).

6. The preparation method according to any one of claims 1 to 5, **characterised in that** said measuring chamber has a volume between 1 cm³ and 12 cm³, in particular between 1.1 cm³ and 9 cm³ and, in particular, of about 1.275 cm³ (i.e. 1,275 cm³ ± 0.05 cm³).

7. The preparation method according to any one of claims 1 to 6, **characterised in that** filling during said step e) is carried out by means of at least two fluidic channels (81, 82) opening into said measuring chamber and adapted to bring, into said measuring chamber, an electrolyte or a precursor of the latter and to extract the air contained in said measuring chamber,
said fluidic channels being perforated at the CO₂-permeable membrane and/or machined at the double-sided adhesive.

8. The preparation method according to any one of claims 1 to 7, **characterised in that** said electrolyte is a liquid electrolyte.

9. The preparation method according to any one of claims 1 to 7, **characterised in that** said electrolyte is an electrolyte in the form of a hydrogel and, advantageously, an agar hydrogel.

10. The preparation method according to any one of claims 1 to 9, **characterised in that** said bicarbonate ions are present, in the liquid electrolyte or in the solution comprising the precursor compounds of the hydrogel forming the electrolyte, in an amount between 1 mM and 20 mM, in particular between 3 mM and 10 mM and, in particular, of about 5 mM (i.e. 5 mM ± 1 mM).

11. A CO₂ sensor obtained by the preparation method according to any one of claims 1 to 10, comprising
- a support **1** of an insulating material having a first face **11** and a second face **12** opposite to the first face, the first face having at least one zone comprising a pH-sensitive electrode **2** and a reference electrode **3**;
- a double-sided adhesive **4** having a first acrylic-based adhesive layer **41**, a second silicone-based adhesive layer **42** opposite to the first adhesive layer and, in its thickness, a through recess **5** having a first open end **51** and a second open end **52** opposite to the first open end;
the first adhesive layer **41** of the double-sided adhesive **4** being in contact with the first face **11** of the support **1** so that the first open end **51** of the recess **5** of the double-sided adhesive **4** is facing the zone of the first face **11** of the support **1**;
- a silicone-based carbon dioxide permeable membrane **6** in contact with the second adhesive layer **42** of the double-sided adhesive **4** and with the second open end **52** of the recess **5** of the double-sided adhesive **4**;
- a measuring chamber **7** the side walls of which correspond to the side edges of the recess **5** of the double-sided adhesive **4**, the lower part of which corresponds to the support **1** of an insulating material at the zone as previously defined and the upper part of which corresponds to the CO₂ permeable membrane **6** covering the second open end **52** of the recess **5** of the double-sided adhesive **4**;
said measuring chamber being filled with an electrolyte **9** comprising bicarbonate ions ( HCO₃⁻).

12. A multi-sensor device comprising at least one CO₂ sensor according to claim 11.

13. A use of a CO₂ sensor according to claim 11 or a multi-sensor device according to claim 12 to measure (i) CO₂ dissolved in a liquid medium in contact with said sensor or said multi-sensor device or (ii) CO₂ gas present in a gaseous fluid in contact with said sensor or said multi-sensor device.
